(19) Europäisches Patentamt
European Patent Office
Office européen des brevets

(11) Veröffentlichungsnummer: **0 168 526**
**B1**

(12) # EUROPÄISCHE PATENTSCHRIFT

(45) Veröffentlichungstag der Patentschrift: **25.01.89**

(51) Int. Cl.⁴: **C 11 D 3/386**, C 11 D 3/12, C 11 D 3/22

(21) Anmeldenummer: **84114491.8**

(22) Anmeldetag: **29.11.84**

(54) **Zur Verwendung in pulverförmigen Waschmitteln geeignete Emzymzubereitung.**

(30) Priorität: **07.12.83 DE 3344104**

(43) Veröffentlichungstag der Anmeldung: **22.01.86 Patentblatt 86/4**

(45) Bekanntmachung des Hinweises auf die Patenterteilung: **25.01.89 Patentblatt 89/4**

(84) Benannte Vertragsstaaten: **AT BE CH DE FR GB IT LI LU NL**

(56) Entgegenhaltungen:
**DE-A-2 531 961**
**DE-A-2 736 903**
**FR-A-2 160 661**
**US-A-4 176 079**

(73) Patentinhaber: **Henkel Kommanditgesellschaft auf Aktien, Postfach 1100 Henkelstrasse 67, D-4000 Düsseldorf- Holthausen (DE)**

(72) Erfinder: **Witthaus, Martin, Dr., Burgmüllerstrasse 7, D-4000 Düsseldorf (DE)**
Erfinder: **Pawelczyk, Hubert, Dr., Alt Eller 23, D-4000 Düsseldorf (DE)**
Erfinder: **Weis, Albrecht, Dr., Amselweg 8, D-4006 Erkrath (DE)**
Erfinder: **Carduck, Franz- Joseph, Dr., Landstrasse 18, D-5657 Haan (DE)**

**EP 0 168 526 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Enzyme, insbesondere Proteasen, finden ausgedehnte Verwendung in Wasch- und Waschhilfsmitteln. Üblicherweise kommen die Enzyme dabei nicht als Konzentrate, sondern in Mischungen mit einem Verdünnungs- und Trägermaterial zum Einsatz. Mischt man solche Enzymzubereitungen üblichen Waschmitteln bei, so kann beim Lagern ein erheblicher Abbau der Enzymaktivität eintreten, insbesondere wenn bleichaktive Verbindungen zugegen sind. Das Aufbringen der Enzyme auf Trägersalze unter gleichzeitiger Granulation (vergleiche DE-A-1 617 190) bzw. durch Aufkleben mit nichtionischen Tensiden (DE-C-1 617 118) oder wäßrigen Lösungen von Celluloseethern (DE-A-1 787 568) führt nicht zu einer nennenswerten Verbesserung der Lagerstabilität, da sich die empfindlichen Enzyme in solchen Aufmischungen regelmäßig auf der Oberfläche der Trägersubstanz befinden. Zwar kann die Lagerstabilität der Enzyme wesentlich erhöht werden, wenn man die Enzyme mit dem Trägermaterial umhüllt bzw. in dieses einbettet und anschließend durch Extrudieren, Pressen und Marumerisieren in die gewünschte Partikelform überführt (vergleiche DE-C-1 617 232, DE-A-2 032 768, DE-A-2 137 042 und DE-A-2 137 043). Derartige Enzymzubereitungen besitzen jedoch nur mangelhafte Löslichkeitseigenschaften, d.h. sie geben bei der Textilwäsche in programmierten Waschmaschinen bei Verwendung kalter oder nur mäßig warmer Waschlaugen den Wirkstoff nicht oder nur zum Teil frei. Die ungelösten Partikel können sich im Waschgut verfangen und dieses verunreinigen bzw. sie werden ungenutzt in das Abwasser überführt. Einbettungsmittel, die aus einem Gemisch fester Säuren bzw. saurer Salze und Carbonaten bzw. Bicarbonaten bestehen (DE-A-1 803 099) und bei Wasserzusatz zerfallen, verbessern zwar das Lösungsvermögen, sind aber ihrerseits sehr empfindlich gegen Feuchtigkeit und erfordern daher zusätzliche Schutzmaßnahmen.

Ein weiterer Nachteil der vorgenannten Zubereitung ist darin zu sehen, daß die Enzyme nur in Form trockener Pulver verarbeitet werden können. Die üblicherweise bei der Enzymherstellung anfallenden Fermentbrühen lassen sich in dieser Form nicht einsetzen, sondern müssen zuvor entwässert werden. An diese Voraussetzung sind auch solche Verfahren gebunden, bei denen leicht lösliche Trägermaterialien, wie Zucker, Stärke und Celluloseether als Bindemittel zur Herstellung von Enzymzubereitungen eingesetzt werden (vergleiche DE-A-2 831 778).

Stärke bzw. wasserlösliche Polymere als Bindemittel sowie feinteilige Zeolithe enthaltende Agglomerate sind aus DE-A-2 736 903 bekannt. Diese Agglomerate werden durch Vermischen des Zeoliths mit dem Bindemittel unter Wasserzusatz hergestellt, dann getrocknet und dann vorgefertigten Waschpulvern zugemischt, in denen sie als Phosphatersatzstoffe fungieren. Eine Lehre des Inhalts, daß die Agglomerate als Träger- und Einschlußmaterial für Enzyme zu verwenden ist, ist der Literaturstelle nicht zu entnehmen. Vielmehr sollen die zeolithhaltigen Agglomerate vorzugsweise keine weiteren Zuschlagstoffe enthalten; lediglich in einigen Fällen soll es zulässig sein, Bleichmittel den Agglomeraten einzuverleiben.

Aus der DE-A-2 531 961 ist ein Verfahren zur Herstellung lagerbeständiger Enzymgranulate bekannt, bei dem die aufkonzentrierte Fermentbrühe mit einem trockenen, feinkristallinen Alkalialumosilikat vom Typ des NaA bzw. NaX vermischt wird. Die erhaltenen Gemische werden anschließend bei Temperaturen bis 50°C, beispielsweise im Wirbelbett getrocknet. Die üblicherweise bei der Enzymverarbeitung austretenden Aktivitätsverluste werden bei diesem Verfahren deutlich reduziert. Da es sich jedoch sowohl bei den betreffenden Enzymen wie auch bei den trockenen, feinkristallinen Alumosilikaten um sehr feinkörnige Pulver handelt, sind bei dem Einarbeiten der Granulate zu Waschmitteln, insbesondere beim Transport zu den Mischvorrichtungen und beim Vermischen mit vorgefertigen Waschmittelgranulaten besondere Vorkehrungen erforderlich, um eine Staubbildung zu verhindern. Es bestand daher die Aufgabe, die Pulvereigenschaften der bekannten Produkte noch weiter zu verbessern und insbesondere die Neigung zur Staubbildung durch eine geeignete Führung des Granulationsprozesses zu vermindern, ohne eine Verschlechterung der Löslichkeit bzw. Dispergierbarkeit der Inhaltsstoffe in kalten bzw. mäßig warmen Waschlaugen in Kauf nehmen zu müssen. Ein weiteres Ziel bestand darin, die Aktivitätsverluste bei der Enzymverarbeitung noch weiter zu vermindern und die Lagerbeständigkeit der Enzyme noch weiter zu erhöhen. Diese Aufgabe wird durch die nachfolgend geschilderte Erfindung gelöst.

Gegenstand der Erfindung ist eine zur Verwendung in Waschmitteln geeignete granulare Enzymzubereitung, bestehend aus Granulaten mit einer Korngröße von 0,1 bis 2 mm, wobei der Anteil der Partikel mit einer Korngröße von unter 0,1 mm nicht mehr als 0,2 Gew.-% beträgt und die Granulate, auf Feststoffe bezogen, wie folgt zusammengesetzt sind:

a) 5 - 25 Gew.-% einer durch Aufkonzentrieren einer Fermentationsbrühe gewonnenen Protease und/oder Amylase, als Trockensubstanz,

b) 10 - 60 Gew.-% eines synthetischen feinkristallinen, gebundenes Wasser enthaltenden Zeoliths vom Typ NaA und/oder NaX,

c) 10 - 50 Gew.-% einer in Wasser quellfähigen Stärke,

d) 5 - 50 Gew.-% mindestens eines in Wasser löslichen Granulierhilfsmittels aus der Klasse der Salze der Carboxymethylcellulose und der Polyethylenglykole,

e) 0 - 15 Gew.-% eines die Zerfallsgeschwindigkeit der Granulate in Wasser steigernden anorganischen Salzes.

Vorzugsweise enthalten die Enzymzubereitungen, auf Feststoffe bezogen, 10 bis 20 Gew.-% der Komponente

(a), 15 bis 45 Gew.-% der Komponente (b), 20 bis 40 Gew.-% der Komponente (c) und 5 bis 30 Gew.-% der Komponente (d), wobei die Summe der Komponente (b) und (c) maximal 75 Gew.-% beträgt. Der Anteil der staubförmigen Partikel mit einer Korngröße von unter 0,1 mm soll vorzugsweise nicht mehr als 0,05 Gew.-% betragen. Die Produkte weisen eine von der Herstellungsweise abhängige Restfeuchte von 3 bis 12 % auf. Dieser Anteil fällt nicht unter die Definition "Feststoffe".

Als Enzyme (Komponente a) kommen in erster Linie die aus Mikroorganismen, wie Bakterien und Pilzen, gewonnenen Proteasen sowie ihre Gemische mit Amylasen in Frage. Sie werden in bekannter Weise durch Fermentationsprozesse gewonnen.

Geeignete Ausgangsstoffe für die erfindungsgemäßen granularen Enzymzubereitungen sind wäßrige Lösungen exocellulärer Enzyme. So können Lösungen von neutralen Proteasen, alkalischen Proteasen oder von Amylasen verarbeitet werden, wobei alkalische Proteasen und deren Mischungen mit Amylasen besonders bevorzugt sind. Unter den alkalischen Proteasen sind solche aus Bacillus-Arten bevorzugt. Es sei hier verwiesen auf L. Keay et al., Biochemical and Biophysical Research Communications Vol. 34 (5), 600 (1969). So können insbesondere Lösungen von Proteasen und/oder Amylasen die aus Stämmen der Gattung Bacillus subtilis, Bacillus licheniformis, Bacillus pumelis, Bacillus subtilis var. amylosacchariticus oder Bacillus amyloliquefaciens eingesetzt werden. Als Ausgangsmaterial für die erfindungsgemäßen granularen Enzymzubereitungen sind Lösungen von Subtilisin Carlsberg oder Subtilisin BPN', sowie Mischungen der beiden, gewünschtenfalls in Gegenwart von Amylasen besonders bevorzugt. Die genannten Substanzen sind ihrer Struktur nach bekannt. So findet sich eine peptidchemische Beschreibung von Subtilisin Carlsberg und Subtilisin BPN' bei E.L. Smith et al. Biol. Chem. Vol. 213 (9), 2184 (1968).

Die für die Verwendung in Waschmittel bevorzugten Enzyme werden üblicherweise durch Züchten geeigneter Stämme der zuvor genannten Bacillus-Gattungen gewonnen. Geeignete Stämme sind beispielsweise in den folgenden Patentanmeldungen beschrieben, und mit Hinterlegungsnummer näher bezeichnet:

DE-1 940 488, DE-2 018 451, DE-2 044 161, DE-2 201 803,
DE-2 121 397, US-3 623 957, US-4 264 738 und EP-6 638.

Erfindungsgemäß ist es möglich, die bei diesen Fermentationsprozessen anfallenden Brühen nach Abtrennen der inaktiven Begleitstoffe, beispielsweise durch Filtration, sowie nachfolgende Aufkonzentration, beispielsweise durch Ultrafiltration und Dünnschichtverdampfung bzw. Gefrierkonzentrierung, unmittelbar in lagerbeständige Granulate zu überführen. Die Entstehung unerwünschter Enzymstäube und die bei zusätzlichen Trocknungsprozessen auftretenden Aktivitätsverluste werden somit vermieden. Darüber hinaus können aber auch andere bekannte Enzyme, wie sie z. B. in der DE-A-2 531 961 offenbart sind, als Bestandteile der erfindungsgemäßen Enzymzubereitungen verwendet werden, wobei auf die zitierte Patentschrift ausdrücklich Bezug genommen wird.

Die als Komponente (b) vorliegenden synthetischen, feinkristallinen Zeolithe vom Typ NaA bzw. NaX lassen sich durch die folgenden Summenformel wiedergeben:

$$0{,}7 - 1{,}1\ Na_2O - Al_2O_3 - 1{,}3 - 3{,}3\ SiO_2 - xH_2O.$$

Diese Zeolithe weisen wechselnde Mengen an chemisch und adsorptiv gebundenem Wasser auf. Dieser Wassergehalt beträgt im allgemeinen 15 bis 25 Gew.-%. Geeignete synthetische Zeolithe stellen feinteilige Pulver dar, die zu mehr als 80 Gew.-% aus Teilchen mit einer Größe von 8 bis 0,1 Mikrometer bestehen. Sie weisen, auf wasserfreie Aktivsubstanz bezogen, ein Calciumbindevermögen von mindestens 100 mg CaO/g auf und können in Waschmitteln Phosphate ersetzen. Sie sind im übrigen in der zitierten DE-A-2 531 961 näher beschrieben, auf die auch an dieser Stelle ausdrücklich Bezug genommen wird. Die unlöslichen Alumosilikate erhöhen überraschenderweise die Zerfalls- und Dispersionsgeschwindigkeit der Teilchen in kaltem und mäßig warmem Wasser bzw. in der Waschflüssigkeit.

Als weiteres Bindemittel (Komponente c) wird in Wasser quellbare Stärke verwendet. Geeignet sind z. B. aus Getreidearten, wie Reis, Mais, Roggen oder Weizen sowie aus Kartoffeln gewonnene Stärkearten. Als gut geeignet hat sich insbesondere Maisstärke erwiesen. Überraschend wurde gefunden, daß die Stärke die Lagerbeständigkeit der Enzymzubereitungen erheblich erhöht.

Weiterhin enthalten die Enzymzubereitungen mindestens ein Granulierhilfsmittel, (Komponente d), aus der Klasse der Salze Carboxylmethylcellulose, insbesondere Natriumcarboxymethylcellulose, und der Polyethylenglykole, wobei letztere ein Molekulargewicht von 1000 bis 20 000, vorzugsweise von 2000 bis 15 000 aufweisen können. Die Natriumcarboxymethylcellulose verbessert die Granulierfähigkeit der Gemische, setzt jedoch in größeren Mengen die Zerfalls- und Dispergiergeschwindigkeit in kalten Waschlaugen herab. Druch einen Zusatz von Polyethylenglykol wird diese Wirkung wieder weitgehend aufgehoben bzw. in Richtung auf eine noch höhere Auflösungsgeschwindigkeit verbessert. Gleichzeitig wirkt dieser Zusatz als Gleitmittel und erleichtert die mechanische Verarbeitbarkeit der Gemische, insbesondere beim Extrudieren sowie beim nachfolgenden Sphäronisieren der Granulate. Ein Gehalt von 5 bis 20 Gew.-% an Natriumcarboxylmethylcellulose und von 3 bis 10 Gew.-% an Polyethylenglykol hat sich als vorteilhaft erwiesen.

Als weitere fakultative Bestandteile können die Granulate noch 0 bis 15 Gew.-% eines die

Zerfallgeschwindigkeit der Granulate in Wasser steigernden anorganischen Salzes enthalten, da so ausgewählt ist, daß es die Lagerbeständigkeit der Enzyme nicht nachteilig beeinflusst. Diese Salze können die Zerfalls- und Dispergiergeschwindigkeit der Granulate in kalten Waschlaugen noch weiter verbessern. Geeignete Zuschläge sind z. B. Natriumsulfat, Natriumchlorid, Natriumhydrogenphosphat, Magnesiumsulfat, Calciumphosphat, Calciumcarbonat oder Bariumsulfat. Als gut geeignet hat sich Natriumsulfat in Mengen von 0,5 bis 3, insbesondere 1 bis 2 Gew.-% bzw. Calciumcarbonat in Mengen von 0,5 bis 15 Gew.-%, vorzugsweise 1 bis 10 Gew.-% erwiesen. Weiterhin können Farbstoffe bzw. Pigmente zwecks Überdeckung der Eigenfarbe der Enzymgranulate anwesend sein, insbesondere Titandioxid, das in Anteilen bis zu 15 Gew.-% eingesetzt werden kann.

Die Gesamtmenge der Komponenten (b) bis (d), also an Zeoltih, Stärke sowie Carboxymethylcellulose und/oder Polyethylenglykol soll vorzugsweise nicht mehr als 80 Gew.-% der Granulate betragen. Im übrigen hat es sich als zweckmäßig erwiesen, den Aktivstoffgehalt der konzentrierten Fermentbrühen sowie den Anteil der Zuschlagsstoffe so zu wählen, daß die Enzymaktivität der Zubereitungen vorzugsweise mindestens 100 000 PE/g (Proteaseeinheiten pro Gramm) enthält.

Zur Herstellung der erfindungsgemäßen Enzymgranulate geht man von Fermentbrühen aus, die - beispielsweise durch Filtration - von den inaktiven Begleitstoffen befreit und in an sich bekannter Weise, beispielsweise durch Ultrafiltration und/oder Dünnschichtverdampfung, auf einen Gehalt an Trockensubstanz von 30 bis 70 Gew.-%, vorzugsweise von 45 bis 60 Gew.-% aufkonzentriert werden. Dieses Konzentrat wird zweckmäßigerweise einem zuvor hergestellten trockenen, pulverförmigen bis körnigen Gemisch der Zuschlagstoffe zudosiert. Zur Herstellung dieses Vorgemisches der Zuschlagstoffe kann der Zeolith entweder als Pulver oder als feuchter, krümeliger Filterkuchen eingesetzt werden. Zur Herstellung der erwünschten krümelförmigen Mischungen empfiehlt es sich, bei Verwendung wasserreicherer Fermentbrühen den Zeolith in trockener, pulverförmiger Form einzusetzen. Insgesamt soll der Wassergehalt der Mischung so gewählt werden, daß sie sich bei der Bearbeitung mit Rühr- und Schlagwerkzeugen in körnige, bei Raumtemperatur nicht klebende Partikel überführen und bei Anwendung höherer Drücke plastisch verformen und extrudieren läßt.

Das rieselfähige Vorgemisch wird anschließend in einem Kneter sowie einem angeschlossenen Extruder zu einer plastischen Masse verarbeitet, wobei als Folge der mechanischen Bearbeitung sich die Masse auf Temperaturen zwischen 40° und 60°, vorzugsweise 45° bis 55°C erwärmt. Das den Extruder verlassende Gut wird durch eine Lochscheibe mit nachfolgendem Zerhacker geführt und dadurch zu zylinderförmigen Partikeln definierter Größe zerkleinert. Zweckmäßigerweise beträgt der Durchmesser der Bohrungen in der Lochscheibe 0,4 bis 1 mm, vorzugsweise 0,6 bis 0,8 mm. Die in dieser Form vorliegenden Partikel können anschließend getrocknet und der späteren Verwendung zugeführt werden. Es hat sich jedoch als vorteilhaft erwiesen, die den Extruder und Zerhacker verlassenden Partikel zuvor zu sphäronisieren, d.h. sie in geeigneten Vorrichtungen abzurunden und zu entgraten. Ein solches Verfahren ist beispielsweise in der DE-A-2 137 042 und der DE-A-2 137 043 näher beschrieben. Man verwendet hierzu eine Vorrichtung, die aus einem zylindrischen Behälter mit stationären, festen Seitenwänden und einer bodenseitig drehbar gelagerten Reibplatte bestehen, wobei die Reibplatte mit einer Geschwindigkeit von 500 bis etwa 2000 Umdrehungen pro Minute rotiert. Vorrichtungen dieser Art sind unter der Warenbezeichnung Marumerizer® in der Technik verbreitet.

Es hat sich als zweckmäßig erwiesen, das den Extruder verlassene Gut mit einem feinteiligen Adsorptionsmittel zu bestäuben, um ein Kleben der Partikel zu vermeiden. Geeignete Adsorptionsmittel sind z. B. Calciumcarbonat, Magnesiumsilikat, Kieselsäureaerogel und Zeolith. Die gleiche Behandlung kann auch während der Sphäronisierung erfolgen.

Nach der Sphäronisierung werden die noch feuchten Kügelchen kontinuierlich oder chargenweise in einer Wirbelschichttrockenanlage bei maximal 40°C Produkt-Temperatur auf ein Restfeuchtegehalt von 3 bis 12, vorzugsweise 6 bis 10 Gew.-% getrocknet. Während dieser Wirbelschichttrocknung können zusätzlich Stoffe zum Umhüllen und Beschichten der Partikel eingebracht werden. Geeignete Hüllstoffe sind wasserlösliche, filmbildende Polymere, insbesondere höhermolekulare, d. h. ein Molekulargewicht von 500 bis 20 000 aufweisende Polyglykole. Weiterhin lassen sich in diesem Stadium auch Farbstoffe und Pigmente auf die Partikel aufbringen, um so eine eventuelle Eigenfarbe, die meist vom Enzymkonzentrat herrührt, zu überdecken bzw. zu verändern. Als inertes und physiologisch unbedenkliches Pigment hat sich insbesondere Titandioxid bewährt, das vorzugsweise in wäßriger Dispersion eingebracht wird. Das über die Pigmentdispersion bzw. über die Polymer-Lösung zugeführte Wasser wird bei der gleichzeitig vorgenommenen Wirbelschichttrocknung wieder entfernt.

Durch nachfolgendes Sieben oder Windsichten werden staubförmige Anteile mit einer Korngröße unter 0,1 mm, vorzugsweise unter 0,2 mm sowie Grobanteile mit einer Korngröße über 2 mm, vorzugsweise über 1,5 mm entfernt und in den Herstellungsprozess zurückgeführt.

Die erhaltene Enzymzubereitung besteht aus weitgehend abgerundeten, staubfreien Partikeln und weist im allgemein eine Enzymaktivität von wenigstens 100 000 PE/g, vorzugsweise von 110 000 bis 130 000 PE/g auf. Sie zeichnet sich, auch im Gemisch mit Wasch- und Reinigungsmitteln sowie Perverbindungen, durch eine sehr hohe Lagerstabilität aus und zerfällt unter Freigabe der inkorporierten Wirkstoffe in kalten besonders in mäßig warmen Waschlaugen innerhalb kurzer Zeit. Sie übertrifft hierin die bekannten konfektionierten Enzymprodukte.

**Beispiel 1**

Eine Fermentbrühe, die durch Fermentation eines mit dem Bacillus licheniformis-Stamm P 300 (vergl. DE-A-2 925 427, hinterlegt unter der Nummer OR 48 am Laboratorium voor Microbiologie, Microbenverzameling, Delft sowie bei der deutschen Sammlung von Mikroorganismen unter der Hinterlegungsnummer DSM 641) geimpften Nährlösung enthalten worden war, wurde durch Filtration von festen Begleitstoffen befreit und durch Ultrafiltration und Dünnschichtverdampfung auf einen Trockenstoffgehalt von 55 Gew.-% und einer Enzymaktivität 400 000 PE/g aufkonzentriert.

In einem mit rotierenden Schlagwerkzeugen ausgerüsteten Mischer wurde ein trockenes Vorgemisch aus 27 Gewichtsteilen eines synthetischen, mikrokristallinen Zeoliths vom Typ NaA (Wassergehalt 21 %, Korngröße 1 bis 8 µm) mit 28 Gewichtsteilen Maisstärke, 10 Gewichtsteilen Na-Carboxymethylcellulose und 5 Gewichtsteilen Polyethylenglykol (Molekulargewicht 2000) hergestellt. Nach vollständiger Homogenisierung wurden 30 Gewichtsteile des wäßrigen Enzymkonzentrates über Düsen innerhalb eines Zeitraum von 2 min zudosiert und das Gemisch noch 1 min bearbeitet. Das rieselfähige Granulat wurde in einen mit Außenkühler versehenen Kneter überführt und homogenisiert, wobei die Produkttemperatur, bedingt durch die mechanische Bearbeitung auf ca. 45 - 55° C anstieg.

Die plastische Masse wurde in einem nachgeschalteten, mit einer Lochscheibe ausgerüsteten Extruder verstrangt. Der Durchmesser der Lochscheiben-Bohrungen betrug 0,7 mm. Die in eine Unterdruckkammer austretenden, eine Temperatur von 50 - 60° C aufweisenden Stränge wurden mittels eines rotierenden Messers auf eine Länge von 0,7 bis 1 mm zerschnitten und mittels einströmender Kaltluft gekühlt und gleichzeitig mit 0,5 Gewichtsteilen Calciumcarbonat-Pulver bestäubt, um ein Verkleben der Partikel zu verhindern. Das Zerkleinern der Stränge bei geringem Unterdruck vermeidet gleichzeitig das Ausbreiten von Enzymstaub.

Die zylinderförmigen Granulate wurden in eine Sphäronisierungs-Vorrichtung (Marumerizer®) überführt und während einer Bearbeitungszeit von durchschnittlich 5 Minuten zu abgerundeten Partikeln verformt, wobei zur Vermeidung des Verklebens 8 Gewichtsteile Calciumcarbonat-Pulver eindosiert wurden. Das den Sphäronisator verlassende Gut wurde kontinuierlich einem Wirbelschichttrockner zugeführt. Im Bereich der Sphäronisierungsstufe herrschte ebenfalls Unterdruck, wobei der entstehende, staubförmige Abrieb mit der strömenden Luft abgeführt wurde.

Im Wirbelschichttrockner wurde das Granulat auf einen Wassergehalt von 5 Gewichtsprozent bei einer Produkttemperatur von maximal 40° C getrocknet. Bei der nachfolgenden Siebung erfolgte die Abtrennung der 1,6 mm übersteigenden, bzw. 0,2 mm unterschreitenden Grob- und Feinanteile. Die abgetrennten Anteile wurden zusammen mit den aus der Abluft isolierten Feinstäuben gesammelt und im Feststoffmischer den pulverförmigen Zuschlagstoffen zugemischt.

Das Produkt enthält 16,5 Gew.-% Enzymtrockenmasse, 27 Gew.-% Zeolith, 28 Gew.-% Maisstärke, 10 Gew.-% Carboxymethylcellulose und 5 Gew.-% Feuchtigkeit.

Eine Bestimmung der Enzymaktivität ergab, daß diese, bezogen auf die Menge und den Aktivstoffgehalt des eingebrachten Enzymkonzentrats, während der Verarbeitung nur um 2,7 % abgenommen hatte. Im Rahmen von Vergleichsversuchen nach bekannten Verfahren hergestellte Granulate bekannter Zusammensetzung, erlitten bei der Verarbeitung erheblich höhere Enzymverluste.

Das erhaltene Granulat bestand aus abgerundeten Partikeln von kugelförmiger bis länglicher Gestalt mit einer Korngröße von 0,2 bis 1,6 mm und einem Staubanteil von weniger als 0,05 Gew.-%. Sie waren nichtklebend, gut schüttfähig und neigten in Aufmischungen mit sprühgetrockneten Waschmitteln nicht zum Entmischen. Hinsichtlich ihrer Lagerbeständigkeit sowie ihrer Lösungseigenschaften übertrafen sie, wie nachfolgend gezeigt wird, vergleichbare Zubereitungen bekannter Zusammensetzung.

Die Lösungsgeschwindigkeit der Granulate wurde wie folgt bestimmt.

In einem Becherglas mit einem Fassungsvermögen von 250 ml wurden 100 ml Leitungswasser (16 °dH = 160 mg CaO/l) bei 20° C mittels eines motorisch angetriebenen Magnetrührers mit einer konstanten Geschwindigkeit von 300 U/min gerührt. Die Länge des Rührstabes betrug 4 mm.

In dem Bereich des nach unten gerichteten Wasserkegels wurde 1 g Enzymgranulat so eingegeben, daß eine Klumpenbildung unterblieb. Nach 20, 40, 60, 90 und 120 Sekunden wurden Proben entnommen und deren Enzymaktivität nach dem Abfiltrieren noch ungelöster Anteile analytisch bestimmt. Angegeben wird der Mittelwert aus 3 Parallelversuchen.

Die nach Beispiel 1 hergestellten Enzymgranulate waren innerhalb 40 Sekunden zu mehr als 50 %, innerhalb 90 Sekunden zu 95 % und innerhalb 120 Sekunden zu 100 % gelöst.

Zur Bestimmung der Lagerbeständigkeit wurden die Proben in Kartonbehälter aus unkaschierter Pappe abgefüllt und im Klimaschrank unter folgenden Bedingungen gelagert.

a) 20° C und 80 % relative Luftfeuchtigkeit
b) 40° C und 50 % relative Luftfeuchtigkeit
c) 40° C und 80 % relative Luftfeuchtigkeit.

Die Proben (a) hatten innerhalb eines 1/2 Jahres keinen Aktivitätsverlust erlitten. Die Halbwertszeit betrug bei den Proben (b) 700 Tage und den Proben (c) 450 Tage. In einer zweiten Versuchsreihe wurden jeweils 2 g Probenmaterial mit 98 g eines marktüblichen Vollwaschmittels (Perboratgehalt 18 Gew.-%) vermischt und unter gleichen Bedingungen getestet. In diesem Fall lag die Aktivität der Probenreihe (a) nach 26 Wochen noch bei

100 %. Die Halbwertszeit betrug bei der Probenreihe (c) 14 Tage.

Die Zusammensetzung weiterer Granulate, die in gleicher Weise hergestellt wurden, ist der Tabelle I zu entnehmen. Die Abkürzungen CMC und PEG bedeuten Carboxymethylcellulose und Polyethylenglykol. Die Zahlenwerte sind Gewichtsprozente. In den Beispielen 8, 9 und 10 wurden 5 Gew.-% des Zeoliths anstelle von Calciumcarbonat zum Pudern der feuchten Granulate verwendet. Das Titandioxid wurde in Form einer 35 %-igen wäßrigen Dispersion während der Wirbelschichttrocknung auf das Granulat aufgesprüht, wobei zur Vermeidung einer Agglomerierung der Granulate dafür gesorgt wurde, daß die Temperatur der Abluft bei Verlassen des Trockners 35° C nicht unterschritt.

Der Verlust an Enzymaktivität während des Verarbeitungsprozesses lag in allen Fällen unter 3 %. Die Korngröße lag zwischen 0,2 und 1,6 mm bei einem Staubanteil von unter 0,05 Gew.-%.

**Tabelle 1**

| Beispiel | Enzym | Zeolith | Stärke | CMC | PEG | sonstige Zusätze | Restfeuchte |
|---|---|---|---|---|---|---|---|
| 2 | 18,0 | 20,0 | 36,0 | 9,0 | 5,0 | 7,3 $CaCO_3$ | 4,7 |
| 3 | 15,0 | 20,0 | 34,0 | 10,0 | 6,0 | 5,0 $CaCO_3$, 5,0 $TiO_2$ | 5,0 |
| 4 | 16,5 | 27,0 | 28,0 | 10,0 | 5,0 | 8,7 $CaCO_3$ | 5,8 |
| 5 | 15,0 | 27,0 | 27,0 | 10,0 | 6,0 | 5,0 $CaCO_3$, 5,0 $TiO_2$ | 5,0 |
| 6 | 17,0 | 28,0 | 28,0 | 10,0 | 4,0 | 8,2 $CaCO_3$ | 4,8 |
| 7 | 18,0 | 28,0 | 29,0 | 10,0 | 3,0 | 7,0 $CaCO_3$ | 5,0 |
| 8 | 17,0 | 31,0 | 26,0 | 10,0 | 3,0 | 7,8 $TiO_2$ | 5,2 |
| 9 | 17,0 | 31,0 | 27,0 | 10,0 | 2,0 | 7,9 $TiO_2$ | 5,1 |
| 10 | 17,0 | 31,0 | 28,0 | 10,0 | 1,0 | 8,0 $TiO_2$ | 5,0 |

Die nach den Angaben in Beispiel 1 durchgeführte Bestimmung der Lösungsgeschwindigkeit ergab die in Tabelle I aufgeführten Werte. Hinsichtlich der Lagerbeständigkeit entsprächen die Granulate dem Produkt gemäß Beispiel 1.

| Beispiel | Zeitbedarf (sec) für eine 50 %-ige Auflösung | Zeitbedarf (sec) für eine 100 %-ige Auflösung |
|---|---|---|
| 2 | 64 | 150 |
| 3 | 52 | 140 |
| 4 | 52 | 140 |
| 5 | 48 | 120 |
| 6 | 59 | 150 |
| 7 | 80 | 180 |
| 8 | 60 | 150 |
| 9 | 65 | 150 |
| 10 | 70 | 170 |

## Patentansprüche

1. Zur Verwendung in Waschmitteln geeignete Enzymzubereitung bestehend aus Granulaten mit einer Korngröße von 0,1 bis 2 mm, wobei der Anteil der Partikel mit einer Korngröße von unter 0,1 mm nicht mehr als 0,2 Gew.-% beträgt und die Granulate, auf Feststoffe bezogen, wie folgt zusammengesetzt sind:

a) 5 - 25 Gew.-% einer durch Aufkonzentrieren einer Fermentationsbrühe gewonnenen Protease und/oder Amylase, als Trockensubstanz,
b) 10 - 60 Gew.-% eines synthetischen feinkristallinen, gebundenes Wasser enthaltenden Zeoliths vom Typ NaA und/oder NaX,
c) 10 - 50 Gew.-% einer in Wasser quellfähigen Stärke,
d) 5 - 50 Gew.-% mindestens eines in Wasser löslichen Granulierhilfsmittels aus der Klasse der Salze der Carboxymethylcellulose und der Polyethylenglykole,
e) 0 - 15 Gew.-% eines die Zerfallsgeschwindigkeit der Granulate in Wasser steigernden anorganischen Salzes.

2. Enzymzubereitung nach Anspruch 1, dadurch gekennzeichnet, daß der Anteil der Komponente (a) 10 bis 20 Gew.-%, der Komponente (b) 15 bis 45 Gew.%, der Komponente (c) 20 bis 40 Gew.-%, der Komponente (d) 5 bis 30 Gew.-% und die Summe der Komponenten (b) und (c) maximal 75 Gew.-% und der Anteil der Partikel mit einer Korngröße von unter 0,1 mm nicht mehr als 0,05 Gew.-% beträgt.

3. Enzymzubereitung nach Anspruch 1 bis 2, dadurch gekennzeichnet, daß als Komponente (d) 5 bis 20 Gew.-

% Natrium-Carboxymethylcellulose und 3 bis 10 Gew.-% Polyethylenglykol mit einem Molekulargewicht von 1 000 bis 20 000 anwesend sind.

4. Enzymzubereitung nach Anspruch 1 bis 3, dadurch gekennzeichnet, daß als zusätzliche Komponente (e) Natriumsulfat oder Calciumsulfat in Anteilen von 0,5 bis 15 Gew.-% anwesend ist.

5. Enzymzubereitung nach Anspruch 1 bis 4, dadurch gekennzeichnet, daß es eine Enzymaktivität von mindestens 100 000 PE/g (Proteaseeinheiten/g) enthält.

6. Enzymzubereitung nach Anspruch 1 bis 5, dadurch gekennzeichnet, daß die Granulate mit einem Überzug aus einem wasserlöslichen filmbildenden, gegebenenfalls Farbstoffe oder Farbpigmente enthaltenden Polymeren versehen sind.

7. Verfahren zur Herstellung des Enzymgranulats nach Anspruch 1, dadurch gekennzeichnet, daß man eine von unlöslichen Bestandteilen befreite, auf einen Gehalt an Trockensubstanz von 30 bis 70 Gew.-% aufkonzentrierte Fermentbrühe mit den unter (b) bis (e) aufgeführten Zuschlagstoffen vermischt, mittels mechanischer Vorrichtung in Granulate überführt, diese bei einer 40°C nicht übersteigenden Produkttemperatur mittels strömender Luft auf einen Restfeuchtegehalt von 3 bis 12 Gew.-% trocknet und die eine Korngröße von weniger als 0,2 mm und mehr als 2 mm aufweisenden Anteile abtrennt.

8. Verfahren nach Anspruch 7, dadurch gekennzeichnet, daß man die durch Vermischen der Fermentbrühe mit den Zuschlagstoffen hergestellte plastische Masse extrudiert und zerkleinert, die erhaltenen Körner von unregelmäßiger Gestalt sphäronisiert, trocknet und mit einem gegebenenfalls Farbstoffe bzw. Pigmente enthaltenden Überzug aus einem wasserlöslichen, filmbildenden Polymeren überzieht.

9. Körniges Waschmittel mit einem Gehalt an granularen Enzymzubereitungen gemäß Anspruch 1 bis 6.

**Claims**

1. An enzyme preparation suitable for use in detergents consisting of granulates having a particles size of 0.1 to 2 mm, the proportion of particles below 0.1 mm in size being no more than 0.2 % by weight and the granulates having the following compositions based on solids:

a) 5 to 25 % by weight of a protease and/or amylase obtained by concentration of a fermentation broth as dry matter

b) 10 to 60 % by weight of a synthetic finely crystalline zeolite containing bound water of the NaA and/or NaX type,

c) 10 to 50 % by weight of a starch swellable in water,

d) 5 to 50 % by weight of at least one water-soluble granulation aid from the class of salts of carboxymethyl cellulose and polyethylene glycols,

e) 0 to 15 % by weight of an inorganic salt which increases the disintegration rate of the granulates in water.

2. An enzyme preparation as claimed in claim 1, characterized in that the proportion of component (a) is from 10 to 20 % by weight, that of component (b) from 15 to 45 % by weight and that of component (d) from 5 to 30 % by weight and the sum of components (b) and (c) is at most 75 % by weight, the proportion of particles smaller than 0.1 mm being no more than 0.05 % by weight.

3. An enzyme preparation as claimed in claims 1 and 2, characterized in that 5 to 20 % by weight sodium carboxymethyl cellulose and 3 to 10 % by weight polyethylene glycol having a molecular weight of 1000 to 200,000 are present as component (d).

4. An enzyme preparation as claimed in claims 1 to 3, characterized in that sodium sulfate or calcium sulfate is present in a quantity of 0.5 to 15 % by weight as additional component (e).

5. An enzyme preparation as claimed in claims 1 to 4, characterized in that it contains an enzyme activity of at least 100,000 PU/g (protease units/g).

6. An enzyme preparation as claimed in claims 1 to 5, characterized in that the granulates are provided with a coating of a water-soluble, film-forming polymer optionally containing dyes or pigments.

7. A process for the production of the enzyme granulate claimed in claim 1, characterized in that a fermentation broth freed from insoluble constituents and concentrated to a dry matter content of 30 to 70 % by weight is mixed with the additives mentioned under (b) to (d), the resulting mixture is converted by machine into granulates, the granulates thus obtained are dried by flowing air at a product temperature not exceeding 40°C to a residual moisture content of 3 to 12 % and the particles smaller than 0.2 mm and larger than 2 mm in size are separated off.

8. A process as claimed in claim 7, characterized in that the plastic mass obtained by mixing of the fermentation broth with the additives is extruded and granulated, the resulting granules irregular in shape are spheronized, dried and coated with a coating of a water-soluble film-forming polymer optionally containing dyes and pigments.

9. A particulate detergent containing the granular enzyme preparations claimed in claims 1 to 6.

**Revendications**

1. Préparation d'enzymes appropriée pour l'utilisation dans les détergents consistant en un granulé ayant une taille de grains de 0,1 à 2 mm dans laquelle la fraction de particules ayant une taille de grains inférieure à 0,1 mm ne s'élève pas à plus de 0,2 % et les granulés, rapporté à la matière solide, ont la composition suivante:

a) 5 à 25 % en poids d'une protéase et/ou amylase obtenue par concentration d'un bouillon de fermentation en tant que substance sèche,
b) 10 à 60 % en poids d'une zéolite contenant de l'eau liée, finement cristallisée, synthétique du type NaA et/ou NaX,
c) 10 à 50 % en poids d'un amidon susceptible de gonfler dans l'eau,
d) 5 a 50 % en poids d'au moins un agent auxiliaire de granulation choisi dans le groupe des sels de carboxyméthylcellulose et des polyéthylèneglycols,
e) 0 à 15 % en poids d'un sel minéral qui augmente la vitesse de décomposition du granulé dans l'eau.

2. Préparation d'enzymes selon la revendication 1, caractérisée en ce que la fraction du composant a) s'élève à 10 à 20 % en poids, celle du composant b) 15 à 45 % en poids, celle du composant c) 15 a 45 % en poids, celle du composant d) 5 à 30 % en poids et celle de la somme des constituants b) et c) au maximum 75 % en poids et le pourcentage de particules ayant une taille de grains de moins de 0,1 mm n'est pas supérieure à 0,05 % en poids.
3. Préparation d'enzymes selon les revendications 1 à 2, caractérisée en ce que comme composant d) 5 à 20 % du sel de sodium de carboxyméthylcellulose et 3 à 10 % en poids d'un polyéthylèneglycol ayant un poids moléculaire de 1000 à 20.000, sont présents.
4. Préparation d'enzymes selon les revendications 1 à 3, caractérisée en ce que en tant que composants additionnels e) du sulfate de sodium ou du sulfate de calcium en quantités de 0,5 à 15 % en poids, sont présents.
5. Préparation d'enzymes selon les revendications 1 à 4, caractérisée en ce qu'elle renferme une activité enzymatique d'au moins 100.000 UP (unités protéasiques)/g.
6. Préparation d'enzymes selon les revendications 1 à 5, caractérisée en ce que les granulés sont pourvus d'un recouvrement à base de polymères solubles dans l'eau, filmogènes, contenant éventuellement des colorants ou des pigments colorés.
7. Procédé d'obtention du granulé d'enzymes selon la revendication 1, caractérisé en ce que l'on mélange un bouillon de fermentation concentré jusqu'à une teneur en substance sèche de 30 à 70 % en poids et débarassé des constituants insolubles, avec les substances additionnelles mentionnées sous b) à d), l'on transforme en granulé au moyen d'un dispositif mécanique, l'on sèche à une température de produit qui ne dépasse pas 40°C au moyen d'un courant d'air jusqu'à une teneur résiduelle d'humidité de 3 à 12 % en poids et que l'on sépare les fractions qui possèdent une taille de grains de moins de 0,2 mm et de plus de 2 mm.
8. Procédé selon la revendication 7, caractérisé en ce que l'on extrude et l'on réduit en petits morceaux la masse plastique obtenue par mélange du bouillon de fermentation avec les substances additionnelles, que l'on "sphéronise" les grains obtenus d'apparence irrégulière, l'on sèche et l'on recouvre avec une couverture qui contient éventuellement des colorants ou des pigments, à base d'un polymère soluble dans l'eau, filmogène.
9. Détergent en grains ayant une teneur en préparation d'enzymes granulée selon les revendications 1 à 6.